# EUROPEAN PATENT SPECIFICATION

(11) **EP 3 664 617 B1**
(45) Date of publication and mention of the grant of the patent: **22.11.2023**
(21) Application number: 18758691.2
(22) Date of filing: 09.08.2018
(51) Int. Cl.: A01N 63/23, C12N 1/20, C12R 1/07

(54) **BIOPESTICIDE IMPROVEMENT VIA GROUP SELECTION**
BIOPESTIZID-VERBESSERUNG DURCH GRUPPENAUSWAHL
AMELIORATION DES BIOPESTICIDES PAR LA SÉLECTION GROUPEÉ

(30) Priority: 11.08.2017 GB 201712908
(43) Date of publication of application: 17.06.2020
(73) Proprietor: University of Exeter, Exeter, Devon EX4 4QJ (GB)
(72) Inventor: RAYMOND, Ben, Penryn Cornwall TR10 9FE (GB); CRICKMORE, Neil, Brighton Sussex BN1 9RH (GB)
(74) Representative: Appleyard Lees IP LLP
(86) International application number: PCT/GB2018/052269
(87) International publication number: WO 2019/030529

(56) References cited:
- EP-A1- 0 409 438
- US-A- 4 713 241
- US-A- 4 990 332
- LINDA J. GAHAN ET AL: "An ABC Transporter Mutation Is Correlated with Insect Resistance to Bacillus thuringiensis Cry1Ac Toxin", PLOS GENETICS, vol. 6, no. 12, 16 December 2010 (2010-12-16), page e1001248, XP055258154, DOI: 10.1371/journal.pgen.1001248
- YOSHIHARU WAKISAKA ET AL: "Asporogenous Bacillus thuringiensis Mutant Producing High Yields of [delta]-Endotoxin", APPLIED AND ENVIRONMENTAL MICROBIOLOGY, vol. 43, no. 6, 1 June 1982 (1982-06-01), pages 1498-1500, XP055513547, US ISSN: 0099-2240
- CRAWFORD I T ET AL: "Methods for mutagenesis of Bacillus thuringiensis", JOURNAL OF MICROBIOLOGICAL METHODS, ELSEVIER, AMSTERDAM, NL, vol. 11, no. 3-4, 1 July 1990 (1990-07-01) , pages 241-246, XP023699135, ISSN: 0167-7012, DOI: 10.1016/0167-7012(90)90060-J [retrieved on 1990-07-01]
- LEILA MASRI ET AL: "Host-Pathogen Coevolution: The Selective Advantage of Bacillus thuringiensis Virulence and Its Cry Toxin Genes", PLOS BIOLOGY, vol. 13, no. 6, 4 June 2015 (2015-06-04), page e1002169, XP055513566, DOI: 10.1371/journal.pbio.1002169
- CHARLOTTE RAFALUK ET AL: "When experimental selection for virulence leads to loss of virulence", TRENDS IN PARASITOLOGY, vol. 31, no. 9, 1 September 2015 (2015-09-01), pages 426-434, XP055513802, GB ISSN: 1471-4922, DOI: 10.1016/j.pt.2015.06.002
- EMANUELE RIGATO ET AL: "Enhancing effect of phenotype mutational robustness on adaptation in Escherichia coli : ROBUSTNESS ENHANCES ADAPTATION IN E. COLI", JOURNAL OF EXPERIMENTAL ZOOLOGY PART B: MOLECULAR AND DEVELOPMENTAL EVOLUTION, vol. 326, no. 1, 27 November 2015 (2015-11-27), pages 31-37, XP055513815, ISSN: 1552-5007, DOI: 10.1002/jez.b.22662
- Levi T. Morran ET AL: "TEMPORAL DYNAMICS OF OUTCROSSING AND HOST MORTALITY RATES IN HOST-PATHOGEN EXPERIMENTAL COEVOLUTION", EVOLUTION, vol. 67, no. 7, 20 December 2012 (2012-12-20), pages 1860-1868, XP055513583, US ISSN: 0014-3820, DOI: 10.1111/evo.12007

## Description

### Technical Field of the Invention

The invention relates to *Bacillus thuringenesis* strains and their uses, and in particular their use as pesticides. The invention also relates to compositions comprising *Bacillus thuringiensis* strains. The invention additionally relates to methods of passaging microbial pesticides using selection among host pest populations to increase the virulence of the microbial pesticides.

### Background to the Invention

Existing pest control technologies have limitations. The efficacy of microbial pesticides and genetically modified (GM) crops, in common with their chemical or synthetic counterparts, is continually threatened by the evolution of resistance. Some important pests have relatively low susceptibility to existing *Bacillus thuringiensis* (*Bt*) toxins prior to selection, while resistance has arisen in response to the use of microbial *Bt* pesticides in several species of Lepidoptera, especially in the diamondback moth *Plutella xylostella.* Increased resistance to GM crops, in some cases associated with failure of field control, is also beginning to emerge (1, 2).

One strategy for coping with resistance is to rely on the discovery of new toxins. A substantial range of *Bt* strains, possessing very diverse Cry toxins have been isolated and characterized. However, while many toxins are known, there are few good toxins that can match the potency and broad host range of one particular class: the Cry1 group. Most field resistance has been developed in response to these toxins; Cry1 toxins are the most common class of toxin engineered into GM plants (2). If the potency of this class is lost to resistance there are few similarly effective replacements. An alternative approach is therefore to try and modify existing well-characterized proteins to improve toxicity in resistant or tolerant pest insects (*3, 4*). A second, commercially important benefit of modifying currently exploited proteins is that they tend to be easier and cheaper to license than entirely new products.

In response to this preference for modified toxins several companies have used random toxin mutagenesis (`directed evolution') as a means of discovering toxin variants with desirable properties. Older methods of screening mutant libraries rely on a cumbersome combination of bioassays and sequencing. One successful recent approach modified 'phage-display' methods to develop a synthetic *in vitro* screen of mutagenized Cry proteins (*5*)*.* This *in vitro* approach did, however, have the limitation of producing Cry proteins that were sensitive to insect larval gut proteases (*5*). It is also a process tied to a single protein-protein interaction while in general approaches based on protein engineering can only be applied to biocontrol methods based on the transgenic expression of modified proteins. The original and still very successful application of *Bt* was as microbial products based on spores and toxins. Artificial selection approaches to improve biological control products at the organismal level could still be valuable for strain improvement in the traditional biological control sector.

Microbial experimental evolution, while still a small field, has had a major impact on evolutionary biology and has addressed fundamental co-evolutionary, ecological and population genetic questions (*6-9*). Many micro-organisms, including *Bt,* can rapidly acquire substantial beneficial mutations in laboratory conditions (*8, 10, 11*). Experimental evolution offers a mechanism-free solution to overcoming pest resistance, and is potentially applicable to many insect pests and a broad class of pathogens.

Experimental passage of insect viruses has been successfully used to increase the virulence of biocontrol agents and to identity genotypes with improved action against resistant hosts (*12*). The high mutation rate of viruses makes them suitable targets for improvement by selection. One means of increasing the rate at which a bacterial pathogen such as *Bt* will adapt to a resistant or tolerant host is to increase its mutation rate. In evolving bacterial populations, hyper-mutable strains or 'mutators' (*13*) (which possess deficient proof-reading genes) are associated with a large proportion of new beneficial mutations. The theory that an increased mutation rate will confer increased rates of adaptation to resistant hosts has wide support. For instance, mutators are favoured by strong selection generally (*14, 15*) and in coevolutionary arms races (*16*). Mutators have evolved elevated virulence in a model insect host more quickly than wild type strains (*17*). Mutators may be more likely to confer increased adaptability under hard selection, *i.e.* when the phenotype of organisms has to exceed a certain critical value in order for them to reproduce. This type of selection may be prevalent in pathogens, which may have to express a certain threshold level of a virulence factor in order to access hosts (*18, 19*). High mutation rates are expected to be more beneficial in small populations where mutation supply is limited (*18, 19*). *Bt* undergoes very tight population bottlenecks in each round of infection (*20, 21*) so that effective population sizes may be small. Mutators have higher recombination rates (*22*)*,* which can facilitate large scale genetic change. While mutators can acquire deleterious mutations and are expected to be eliminated by long term selection, experimental evolution has shown that they can persist in populations for thousands of generations without being eliminated by reversion or recombination (*19*).

A key recent advance in the understanding of microbial virulence has been the application of 'social evolution' theory, *i.e.* ideas relating to the maintenance of cooperative or altruistic traits (*7, 24, 25*). Micro-organisms engage in a form of cooperative behaviour known as public goods production. Public goods, in economic terms, are goods or services that benefit entire communities but which are costly for individuals. In bacteria this communal availability occurs through long-distance secretion of metabolites into extracellular space. Bacterial public goods include biofilms, non-sporulating components of fruiting bodies, virulence factors and enzymes important for nutrient acquisition (*25-28*). Critically this includes the Cry toxins produced by *Bt,* and to some degree a host of vegetatively produced factors that are regulated by quorum-sensing (21, 28), which are important for enabling these bacteria to invade the insect host. Two crucial factors are important for the maintenance of cooperative virulence: high relatedness (or low diversity within groups) and a population structure that facilitates between group competition (7). Relatedness is critical because with diverse infections competition between genotypes will favour "cheater" mutants that have deactivated costly production of virulence in favour of rapid growth. Population structure, the division of a population into coherent groups facilitates group level selection on traits that are beneficial to groups rather than individuals.

It is an object of the present invention to overcome one or more problems associated with current pest controls. It is also an object to provide an improved pest control. Preferably, the pest control will utilise a microbe, such as *B. thuringiensis* and the strains will have a higher virulence profile. It would be desirable for methods to be developed so as to evolve diverse strains of *B. thuringiensis* and enable selection of the most efficacious/virulent strains from a diverse pool and improve virulence.

US4990332 discloses a novel and useful insecticide with activity against insect pests of the order Lepidoptera. Pests in the order Lepidoptera do heavy damage to crops, e.g., cabbage and broccoli. The insecticide is a novel B. thuringiensis microbe referred to as B.t. PS85A1, or mutants thereof. Specifically disclosed is an asporogenous mutant designated B.t. PS85A1-168. The spores or crystals of B.t. PS85A1, or the crystals of B.t. PS85A1-168, are useful to control lepidopteran pests in various environments.

US4713241 discloses a bacterial insecticide containing an insecticidal substance produced by a practically reversionless asporogenous mutant of Bacillus thuringiensis, especially Bacillus thuringiensis serovar. kurstaki 290-1 and the process for the production in addition to a practically reversionless asporogenous mutant of Bacillus thuringiensis, especially Bacillus thuringiensis serovar. kurstaki 290-1 and the process for the production.

EP0409438 discloses novel Bacillus thuringiensis (B.t.) isolates which have activity against all mosquitoes tested. Parent B.t. isolates are treated to obtain lysis<-> spo<-> cry<+> mutants which retain the biological activity of the parent B.t. isolates.

Linda J. Gahan et al., (2010) (PLOS Genetics, vol. 6, no. 12, page e1001248) discloses a study to identify mutations which may be responsible for a loss of binding of Cry1Ac toxin to the mid-gut membrane. A map-based cloning approach was used to identify a resistance gene in the cotton pest *Heliothis virescens.* An inactivating mutation of the ABC transporter ABCC2 that is genetically linked to Cry1Ac resistance and is correlated with loss of Cry1Ac binding to membrane vesicles was discovered. This weakness could be exploited to manage this mechanism of Bacillus thuringiensis resistance in the field.

Yoshiharu Wakisaka et al., (1982) (Applied and Environmental Microbiology, vol. 43, no. 6, pages 1498-1500) discloses an asporogenous Bacillus thuringiensis subsp. kurstaki strain IK mutant, strain 290-1, which produced high yields of ^{δ}-endotoxin, was obtained by ethyl methane sulfonate treatment of a spore suspension. The mutant strain produced about the same amount of ^{δ}-endotoxin as that produced by the parent strain, but 10¹⁰ to 10¹¹ cells did not form any detectable dormant spores.

Crawford I. T. et al., (1990) (Journal of Microbiological Methods, vol. 11, no. 3-4, pages 241-246) discloses a study for mutagenesis and isolation of initial auxotrophic and sporulation mutants of B. thuringiensis, using both chemical and transposon mutagenesis techniques. Bacillus thuringiensis ssp. kurstaki strains HD-1 and cry-B, an acrystalliferous mutant, could be mutagenized with the chemical ethyl methanesulfonate (EMS) and with the transposon Tn917. Both auxotrophic and sporulation mutants were obtained.

Leila Masri et al., (2015) (PLOS Biology, vol. 13, no. 6, page e1002169) discloses combined experimental evolution of the bacterial biocontrol agent Bacillus thuringiensis and its nematode host Caenorhabditis elegans with large-scale phenotyping, whole genome analysis, and functional genetics to demonstrate the selective benefit of pathogen virulence and the underlying toxin genes during the adaptation process.

Charlotte Rafaluk et al., (2015) (Trends in Parasitology, vol. 31, no. 9, pages 426-434) discloses a critical overview of experimental tests of hypotheses regarding virulence evolution and provides potential explanations for the contradictory results. The key role of parasite transmission mechanisms is described and observed discrepancies among evolution experiments are explained.

Emanuele Rigato et al., (2015) (Journal of Experimental Zoology Part B: Molecular and Developmental Evolution, vol. 326, no. 1, pages 31-37) describes an experiment to investigate the role of robustness in the adaptation of the bacterium Escherichia coli by measuring the effects of CGV in adapting to novel environments, represented by minimal media with a single carbon source, different from the original strain's native carbon source which is glucose. The two phenotypic traits under scrutiny were the instantaneous population growth rate in either glycerol or lactate, which can be interpreted as measures of population absolute fitness in these environments. The results show an enhancing effect of CGV on the process of adaptation to a novel environment, with quantitative differences between the two traits that match an independent measure of trait robustness, based on the survival rate after mutagenesis in the two environments.

### Summary of the Invention

The invention is described in the appended claims.

In accordance with an aspect, there is disclosed a *Bacillus thuringiensis* strain which is phenotypically stable and has increased virulence compared to the wild-type strain, whereby the increased virulence has been achieved by the exposure of the strain to a mutagen during one or more passages wherein said a *Bacillus thuringiensis* strain comprises *Bacillus thuringiensis kurstaki* NCTC 17080301 or *Bacillus thuringiensis kurstaki* NCTC 17080302.

Said *B. thuringiensis* strain has an increased virulence relative to its ancestor. The increase in virulence of the *B. thuringiensis* strain may be at least 10 times that of its ancestor. More preferably the increase in virulence of the *B. thuringiensis* strain may be at least 100 times that of its ancestor.

The mutagen may comprises ethyl methanesulfonate (EMS).

The *B. thuringiensis* strain may be for a non-therapeutic use as a pesticide. The B. *thuringiensis* strain has an increased virulence towards pests relative to its ancestor.

The strains of the present invention have been found to advantageously have increased killing power against target pests, including those pests having at least some degree of pesticide resistance.

The *B. thuringiensis* strain may be used in the control of pests including insects, mites, nematodes and gastropods. The *B. thuringiensis* strain may be used in the control of one or more of the following: Lepidoptera, Diptera, Coleoptera and nematodes.

Preferably the *B. thuringiensis* strain may be used in the non-therapeutic control of one or more of the following: aphids, other Hemipteran pests, thrips, Lepidopteran larvae, Dipteran larvae, Coleopteran larvae, spider mites, locusts, crickets, ants, cockroaches, flies, wasps, termites woodworm, wood ants, bookworms, silverfish, carpet beetles, clothes moths, gypsy moths, chiggers, *Sarcoptes scabiei,* ticks, mites, lice, fleas, bed bugs, mosquitoes, tsetse flies, kissing bugs, root-knot nematode, soybean cyst nematode, potato cyst nematode, slugs and snails.

The *B. thuringiensis* strain may be used in the non-therapeutic control of insects. Preferably the *B*. *thuringiensis* strain is used in the control of lepidopterans. The *B. thuringiensis* strain may be used in the control of diamondback moth *Plutella xylostella* and its larvae.

The *B. thuringiensis* strain may be used to control pests of plants including crops. Preferably the *B. thuringiensis* strain may be used to control pests of *Brassicaceae* plants. Specifically the *B. thuringiensis* strain may be used to control pests of cruciferous plants. The *B. thuringiensis* strain may be used to control pests of horseradish, land cress, Ethiopian mustard, kale, collard greens, Chinese broccoli, cabbage, Savoy cabbage, Brussels sprouts, kohlrabi, broccoli, broccoflower, broccoli romanesco, cauliflower, wild broccoli, bok choy, komatsuna, mizuna, rapini, choy sum, Chinese cabbage, turnip root, rutabaga (swede), Siberian kale, canola/rapeseed, wrapped heart mustard cabbage, mustard seeds, white mustard seeds, black mustard seeds, tatsoi, wild arugula, arugula, field pepperweed, maca, garden cress, watercress, radish, daikon and wasabi.

The *B. thuringiensis* strain may be applied to, near to, or in the soil of plants. The *B. thuringiensis* strain may be applied to the leaves, stems, flowers and/or roots of plants.

The *B. thuringiensis* strain has similar levels of potency to Cry susceptible insects as its ancestors. Effective rates of application against Cry susceptible insects may therefore be in the range of 0.01-0.1 mg of the *B. thuringiensis* strain per cm² of crop surface area or 10,000 to 100,000 of the *B. thuringiensis* strain spores per cm² of crop surface area. Effective rates of application against Cry1A resistant insects will depend on the precise levels of resistance and efficacy of the *B. thuringiensis* strain against that genotype but may be in the range of 0.01-0.5 mg of the *B. thuringiensis* strain per cm² of crop surface area or 10,000 to 500,000 of the *B. thuringiensis* strain spores per cm² of crop surface area.

The a *Bacillus thuringiensis* strains comprise SEQ ID No. 2 or SEQ ID No. 3 or SEQ ID No. 4.

In accordance with a further aspect, there is disclosed a composition comprising a *Bacillus thuringiensis* strain comprising *Bacillus thuringiensis kurstaki* NCTC 17080301 or *Bacillus thuringiensis kurstaki* NCTC 17080302.

The *B. thuringiensis* strain may be added to the composition in the form of spores or crystals.

The composition may be in the form of a solid or a liquid.

The composition may further comprise one or more of the following ingredients: wetting agents, thickeners, viscosity modifying agents, stabilisers and surfactants. The composition may comprise a dispersing medium such as water or edible oils.

The composition may be used or for non-therapeutic use as a pesticide.

The composition may be applied to (or near to) plants and/or the soil surface. Alternatively the composition may be incorporated into the soil itself. The composition may be applied to plants and/or soil by spraying, spreading or sprinkling. The composition may be applied to the leaves, stems, flowers and/or roots of plants.

In a further aspect, there is disclosed a method for improving virulence of microbial pesticides comprising
(a) splitting a population of pests into subpopulations
(b) exposing the subpopulations of pests to one or more microbial pesticides; wherein the microbial pesticide has been exposed to a mutagen and wherein the microbial pesticide comprises a bacterium mutated by inactivation of the mutS gene;
(c) selecting pest cadavers from the subpopulations with highest mortality
(d) inoculating sporulation media with the pest cadavers so as to provide sufficient readily quantifiable inoculum for a subsequent round of infection;
(e) purifying and quantifying microbial spores from the selected cadavers so as to identify a microbial pesticide having improved virulence; and wherein the microbial pesticide is exposed to a mutagen

The method may further comprise using microbial spores from step (e) to infect one or more second pest populations.

Steps (a) to (e) may be repeated by using the purified microbial spores from step (e) to infect one or more pest populations. Steps (a) to (e) may be defined as a passage and the resulting microbes as passaged microbes. Optionally, or preferably, the microbial pesticide is repeatedly exposed to the mutagen at each passage. Alternatively, the microbial pesticide may be exposed to the mutagen at alternate passages or only during the initial passages. The purified spores from step (e) may be used to infect at least 2 pest populations. Preferably steps (a) to (e) are repeated at least once. Steps (a) to (e) may be repeated at least 5 times.

Cadavers may be selected during step (c) on the basis of total mortality of the pest population reaching in the range of 20-40%. Preferably the cadavers are selected during step (c) on the basis of total mortality of the pest population reaching in the range of 25-30%.

The above method provides group level competition on mortality rates in an insect meta-population which advantageously means that pathogens are only selected from groups with high levels of mortality for further passages.

The one or more pest populations may comprise genetically variable populations. This allows for the pathogens to adapt to a hard to kill host. The pest populations may comprise different levels of resistance to the microbial pesticides or one or more toxic compounds produced by the microbial pesticides. The pest populations may comprises an intermediate level of resistance to the microbial pesticides or one or more toxic compounds produced by the microbial pesticides.

The method may be for use in providing a library of microbial pesticides. In certain embodiments, there is disclosed a library of microbial pesticides produced by the above described method. The library may be formed of a plurality of microbial pesticides having increased virulence when compared to the virulence of the wild-type microbial pesticides.

The mutagen may comprise ethyl methanesulfonate (EMS). In the method, the microbial pesticides may be incubated with 0.05 to 1.0 % EMS. The microbial pesticides may be incubated with 0.1 to 0.5 % EMS, with 0.1 to 0.4 % EMS, with 0.1 to 0.3 % EMS, with 0.1 to 0.2 % EMS. Most preferably, the microbial pesticides are incubated with about 0.2 % EMS.

The mutagen is preferably incubated with the selected pest cadavers prior to sporulation media inoculation. Also preferably, the mutagen is substantially removed prior to sporulation media inoculation. Removal may be by a number of means, such as centrifugation.

The purified microbial spores from step (e) may be used as a pesticide.

The microbe may be an insecticide. The microbe may be an insecticide with established pathogenicity. The microbial pesticide may comprise a bacterium. Preferably the bacterium comprises *B. thuringiensis.* The *B. thuringiensis* strain may be *Bacillus thuringiensis entomocidus, B. thuringiensis kurstaki* or *B. thuringiensis aizawai.* Preferably the *B. thuringiensis* strain comprises *B. thuringiensis kurstaki, B. thuringiensis aizawai B. thuringiensis tenebrionis, B. thuringiensis israelensis* or any other insect or nematode pathogenic *B. thuringiensis* strain.

The pests may be insects, mites, nematodes or gastropods. Preferably the pests are aphids, other Hemipteran pests, thrips, Lepidopteran larvae, Dipteran larvae, Coleopteran larvae, spider mites, locusts, crickets, ants, cockroaches, flies, wasps, termites woodworm, wood ants, bookworms, silverfish, carpet beetles, clothes moths, gypsy moths, chiggers, Sarcoptes scabiei, ticks, mites, lice, fleas, bed bugs, mosquitoes, tsetse flies, kissing bugs, root-knot nematode, soybean cyst nematode, potato cyst nematode, slugs and snails.

Preferably the pests are lepidopterans. The pests may be diamondback moths *Plutella xylostella* and its larvae.

The microbe is mutated, preferably by chemical mutagenesis. The microbe is bacterium mutated by inactivation of the *mutS* gene. Advantageously hyper-mutable strains or 'mutators' are associated with a high proportion of new beneficial mutations and an increased mutation rate may confer increased rates of adaptation to resistant hosts. Mutators also have higher recombination rates which can facilitate large scale genetic change.

There is also disclosed a method for screening for improved virulence in microbes comprising:
(a) growing purified microbial pesticide spores;
(b) infecting a pest population; and
(c) recording number of pest deaths.

The pest population may be infected with at least 1 dose of microbial pesticide spores. Preferably the pest population is infected with at least 3 doses of microbial pesticide spores. There may be 10¹-1.2×10⁵ spores µl⁻¹.

### Detailed Description of the Invention

Embodiments of the present invention will now be described, by way of example only, in which:
Figure 1 is a schematic diagram showing the steps involved in infecting insect populations with bacterial spores according to group selection and pool selection methods;
Figure 2 is a bar chart showing early larval mortality of Cry1Ac resistant larvae of *P. xylostella* exposed to ancestral (wt anc), unevolved *B. t. kurstaki* and evolved treatments. Data are means and SEs pooled over 2 replicate bioassays, at three doses for four replicated lineages per treatment;
Figure 3 is a group of 6 graphs showing the overall mortality of Cry1Ac resistant larvae of *P. xylostella* exposed to ancestral (wt anc) unevolved *B. t. kurstaki* and evolved treatments. Data are proportional mortality per dose (*n* = 50-60 larvae) with different symbols for replicate lineages within treatments. Lines are fitted statistical models (back-transformed into probability) fitted for each lineage and each bioassay. Line types indicate (dashed or solid) independent bioassays. Doses are viable spores per microlitre;
Figure 4 is two graphs showing the efficacy of the best performing wild type clone (p3c2) against *B. t. kurstaki* resistant and susceptible *P. xylostella* in comparison to its ancestor and an alternative commercial *Bt* isolate (XenTari) used to target resistant larvae. Data are raw 5 day mortality from bioassays (*n* = 60 larvae per dose);
Figure 5 is two graphs showing the relative fitness of selected evolved clones, p3c1 *(Bacillus thuringiensis kurstaki* NCTC 17080301)) and p3c2 (*Bacillus thuringiensis kurstaki* NCTC 17080302), with increased virulence relative to ancestor across a range of initial frequencies. Data are based on the ratio of estimated Malthusian parameter; dashed line indicates fitted linear models with SE in grey. *N* =40-50 larvae per treatment;
Figure 6 is two graphs showing the data of the Cry1Ac-resistant and -susceptible lavae;
Figure 7 is a graph showing the alternating background clone bioassay in Cry1Ac-susceptible larvae;
Figure 8 is three graphs showing the bioassay of resistant and co-evolved passage treatments for co-evolved larvae, Cry1Ac-resistant larvae and Cry1Ac-susceptible larvae; and
Figure 9 is a graph showing the results of a bioassay assessing the toxin class for the initial mix, HCO and HCO+EMS in the toxin library selection experiment.

### Example

The first experiment sought to increase virulence of *B. t. kurstaki* to an insect host genotype of diamondback moth (DBM) *Plutella xylostella,* which was highly resistant (LC50 ≈ 10⁵ spores) to infection. The supply of mutations was manipulated by evolving wild type and mutator (mut) bacteria. In addition, group level selection was imposed in two ways: the first treatment used pooling of larvae from sub-populations at each round of infection, a regime that should select on high bacterial population size within insects and a protocol that has produced modest increases in virulence previously (*11*). The second selection treatment imposed group-level competition on mortality rates in a meta-population: in each round of selection pathogens were only propagated from groups with high levels of mortality. In the second selection experiment a *Bt* biopestidal isolate (*B. t entomocidus i*) was evolved with already quite effective virulence in *P. xylostella* (LC50 ≈ 10² spores); in this experiment we used a mutagen (EMS) to supply addidtional mutations in lieu of a mutator strain, and varied the insect genetic background used to impose selectin on virulence, in order to either improve evolution of virulence or produce mutants capable of infecting more than one genotype with higher efficacy.

### Strains used and construction

The wild type *B. t. kurstaki* strain was obtained by transforming 7.1.o strain with pHT315-gfp plasmid containing the *ermB* gene conferring erythromycin resistance (*21*) . It was maintained *in vitro* with 10ug/mL erythromycin. When grown from insect cadavers, 300µg/mL erythromycin was used to inhibit growth of Gram-negative bacteria. The mut (mutator) strain was obtained by inactivating the *mutS* gene in 7.1.o. A fragment of *mutS* coding sequence containing an insertion of the *cat* gene was synthesised and cloned within BamHI sites of the thermosensitive vector pRN1501 (*29*). The resulting plasmid was transformed into 7.1.o strain, and clones with recombination of the interrupted *mutS* sequence into the chromosome were obtained after growth at 42°C by screening for chloramphenicol (Cm) resistance and loss of erythromycin resistance (*29*). Insertion was confirmed by PCR and sequencing of *mutS* locus. The mutator phenotype of the resulting mut strain was confirmed with fluctuation tests showing an increase in mutation rate of 40-fold compared to the wt strain. The mut strain was grown in the presence of 5ug/mL Cm. The strain of *B. t. entomocidus* in passage experiment 2 was recovered from a commercial the Bacillus Genetic Stock Centre (BGSC 414) and transformed with pHT315-gfp plasmid containing the *ermB,* as above.

### Passage experiment 1; scale of competition and mutation supply

Diamondback moth larvae used for selection were reared on an autoclaved artificial diet (*30*) without supplementary antibiotics until 3^{rd} instar. *Bt* strains used for the selection experiment were first grown from single colonies into 100 mL of sporulating media (HCO) with appropriate antibiotic, then pasteurised (65°C, 20min). Selection was then performed with 4 replicate lineages per treatment. The Cry1Ac resistant line of *Plutella xylostella* used for experiments was derived from a cross of NOQA-GE (from David Heckel, Max Planck, Jena) with the diet adapted susceptible line Vero Beach (from Oxitec UK), this line was selected for resistance to Cry1Ac and denoted "VL-FR". A population that was fixed for resistance using a PCR screen of resistance alleles in the parental population (*31*) was established. An insect strain with similar genetic background (derived from NOQA-GE X Vero Beach) was established from F2 offspring but in this case fixed for susceptibility to Cry1Ac, this line was denoted 'VL-SS'.

For infection, sterile diet was cut into quarters in 55mL dishes, and each quarter was inoculated with 90uL of spore suspension containing 5×10⁴ to 10⁵ spores/µL (chosen to produce 25-30% mortality over 2-4 days for resistant diamondback moth) and dried. 10 third instar larvae were then added per 55mL dish. Insect death was recorded from two days after infection. When total mortality reached 25-30%, the earliest cadavers were transferred to micro-centrifuge tubes with sterile toothpicks. After two days at room temperature (20-22°C), 0.85% NaCl solution (saline) was added to the tubes. Cadavers were homogenised with pellet pestles, then the tubes were briefly vortexed and the suspension was transferred to wells containing 1 mL of sporulation medium in 24-well growth plates, for spore amplification *in vitro.* Sporulation medium was HCO (32) containing polymyxin (100 IU/mL) (Oxoid) and an additional relevant antibiotic: erythromycin 300 µg/mL for wt lineages containing pHT315:gfp or chloramphenicol 5 µg/mL for mut lineages containing the chromosomal *cat* marker. Rows of inoculated wells from the same line of selection were alternated with empty rows to prevent and control for contamination between lines. The plates were then sealed with tape to limit evaporation, and placed at 30°C with 150rpm agitation for three days.

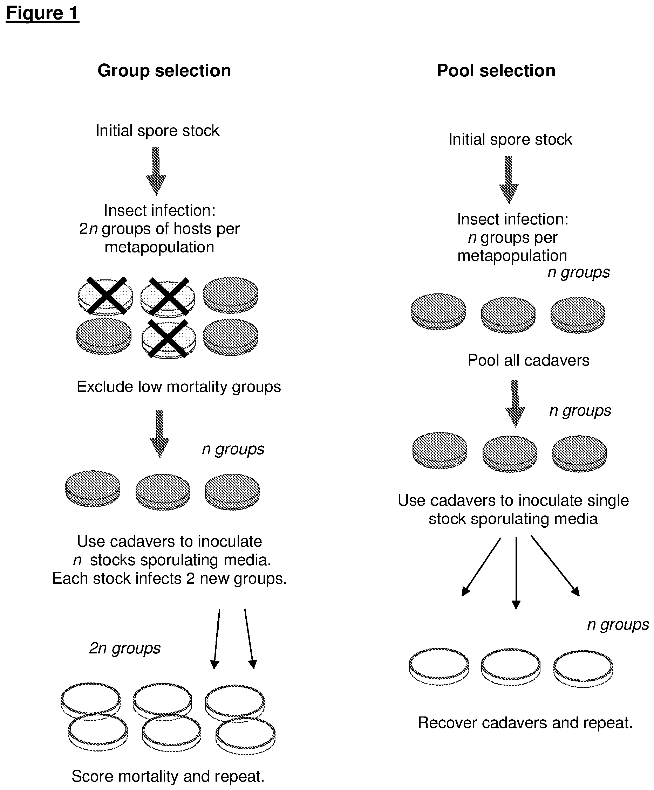

Selection of cadavers and inoculation into sporulation medium depended on experimental treatment (as shown in Figure 1). This experiment compared the efficacy of selecting for virulence by imposing group level competition on mortality between subpopulations in an insect host meta-population (group selection) or by imposing selection on efficient replication of pathogens in pools of cadavers (pool selection). For the group selection treatment, each lineage was split into 12 populations maintained separately. The 6 populations with highest larval mortality were selected; 6 other populations were discarded. If the same mortality was observed for several populations, the ones with earliest observed deaths were selected. Cadavers from selected populations were transferred into separate tubes (6 total). 50 µL saline was added in each tube, and the suspension from each tube was inoculated into separate wells. After spore purification (described below), spores from each well were used to inoculate 2 populations in the next selection round. For the pooled selection treatment, each lineage was split into 6 populations, which were pooled together at each round of selection. All cadavers from 6 populations in the pooled treatment were transferred to the same microfuge tube. 300µL saline was then added and the suspension was distributed into 6 wells for inoculation. To maintain comparable population sizes, at inoculation, across treatments, only a subset of cadavers was selected from replicate lineages that had more than 30% death at time of selection.

After 3 days of growth, 200 µL spore aliquots were transferred to 96-well PCR plates with aluminium sealing film for quantification, infection in next passage and storage. Plates were centrifuged at 3000 g for 15 min, the supernatant was removed and spores were resuspended in 100 µL 0.85% NaCl. Plates containing spores to be used in next infection step were pasteurised (20 min, 65°C) and kept at 10°C until use, the others were stored at - 20°C. Spore density was estimated by diluting 10 µL of spores into 90 µL saline and measuring OD600nm in a 96-well microplate reader. A subset of the samples was plated at appropriate dilutions on LBA plates, in order to visually check for contamination and calibrate OD600nm measurements.

### Phenotypic and genotypic characterization of strains

### Bioassays

After five rounds of selection, the evolved lineages were frozen at -80°C (LB 20% glycerol) without pasteurisation. These frozen stocks were used to inoculate sporulation medium for assays on whole populations. Clones were isolated from population samples by streaking 3 times (successively single colonies on LB agar plates supplemented with 20 µg/mL erythromycin (wt clones) or 5 µg/mL chloramphenicol (mut clones).

For bioassays, mixed populations or clones were inoculated into 1 mL HCO (without antibiotics) and grown for 3 days at 30°C, with 150 rpm agitation. Spore concentration was measured by plating on LBA after pasteurisation.

Lineage-level assays were carried out twice with independent spore stocks. Each assay was performed with at least three doses with 50-60 insects per dose. Initial screens of clone level variation examined 6 clones per lineage; these used 15-30 insects per dose at two doses. Data shown are deaths after 5 days. In passage experiment 1 clonal screens were analysed in terms of viable spores (counted as colony forming units on LBA) but also in terms of dilution factor of inocula relative to purified spore stocks as some clones displayed variable germination rates on LBA. Clones of interest were further characterized in additional bioassays, with methods that followed lineage assays, these assays were repeated at least twice.

### Genotypic and phenotypic characterization

Ten evolved clones from both the wild type and mutator backgrounds, in addition to their respective ancestors were selected for whole genome sequencing on an Illumina HiSeq 2500 at a minimum of 30x coverage by 150bp paired end sequencing in a single multiplexed run. DNA was extracted using Qiagen genomic extraction kits, after appropriate lysis for Gram-positive bacteria. Genomes were assembled using Velvet (*33*) and mapped against draft assemblies of ancestors and the completed assembly of *B. t. kurtaki* HD-1 using PAGIT(*34*). Measurements of relative fitness within insects used a marked strain of the 7.1.o wild type ancestor transformed with a pHT315-rfp plasmid containing the *tetR* gene conferring tetracycline resistance (*21*). This marked ancestor therefore has a plasmid with a similar backbone to the evolved wild type strains but is distinguishable on the basis of its distinct antibiotic resistance. Relative fitness is calculated from an estimate of relative reproductive rates of two genotypes within each insect, as described previously (*21*).

### Results -Passage experiment 1

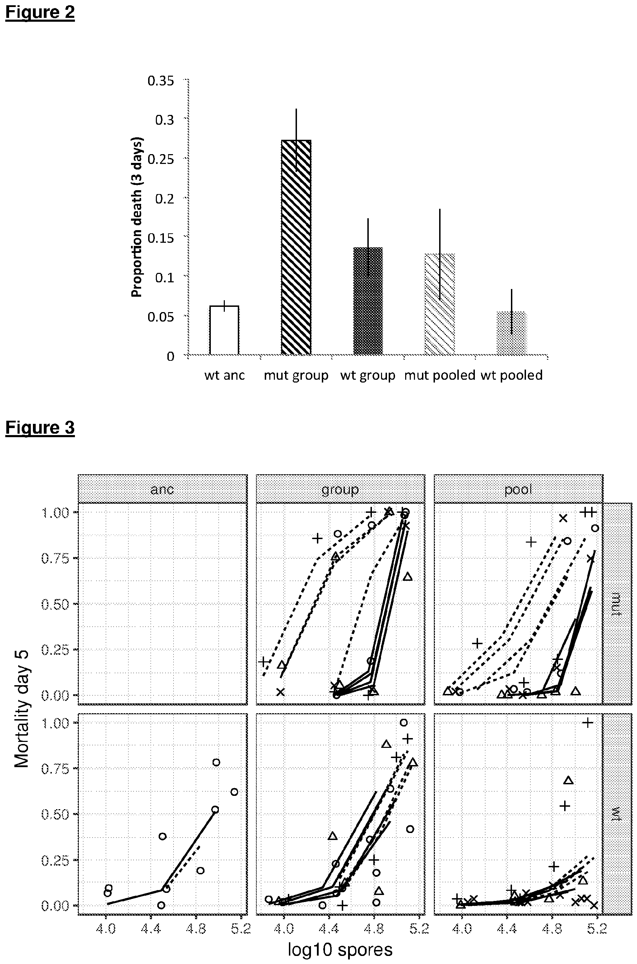

After five rounds of selection clear differences in virulence between selection treatments (pooled and group) and between the two genetic backgrounds (wild type and mut) relative to the ancestral wild type were seen. Figure 2 shows the change in early larval mortality of Cry1Ac resistant larvae of *P. xylostella* exposed to ancestral (wt anc) unevolved *B. t. kurstaki* and evolved treatments. For early mortality, both group level selection (*F*_{1,44} = 6.54, *P* = 0.014) and the mutator background increased the effectiveness of evolved *Bt* lines in killing resistant insects (*F*_{1,45} = 4.10, *P* = 0.049) (see Figure 2). The data from overall (day 5) mortality broadly confirms these simple conclusions. Mutators clearly performed better than wild type evolved strains- doses as low as 25,000 spores were regularly producing mortality in excess of 75%, while 150,000 spores of the ancestor could not reliably produce this level of mortality. Group selection was also more effective than pooled selection, although the gains for wild type lineages were weaker than for mutators (as shown in Figure 3). The full analysis of overall mortality was more complex as the effects of genetic background and selection treatment interacted with bioassay dose. However, pooling cadavers as a selection treatment was clearly inferior to group selection (generalized linear mixed model (glmm), *z* = -2.37, *P* =0.0178) while the mutators were more efficient at killing insects because mortality responded more rapidly with dose (glmm, *z* = -10.67, *P* <0.0001) (see Figure 3). Figure 3 shows the change overall of Cry1Ac resistant larvae of *P. xylostella* exposed to ancestral (wt anc), unevolved *B. t. kurstaki* and evolved treatments.

However, it is important to note that assays conducted with evolved lineages are potentially diverse bacterial assemblages and that lineages with increased virulence may contain clonal variants with substantially higher gains in killing power toward previously resistant insects. To investigate this clonal level variation 6 clones for each evolved lineage from passage 5 were streaked out and characterized as well as 6 clones from a subpopulation of a wild type group selected lineage that showed enhanced virulence at passage 3. The analysis of passage 5 clones broadly confirmed the lineage level assays, in that mutator and group selection treatments identified more clones with putative increases in virulence (as shown in Table 1 below). This analysis also showed that evolved lineages (especially in the pooled treatment) contain a substantial number of clones that appeared to have reduced virulence relative to ancestors (as shown in Table 1).

**Table 1 (Clone level endpoint assays from passage 5. Six clones per independent lineage (48 overall) were screened for increases or decreases in virulence relative to their ancestor in two-dose discriminating assays. These data indicate the number of evolved clones with significant (P < 0.05) changes in virulence in logistic regression models with binomial errors.)**

| Treatment | Clones with increased virulence | Clones with decreased virulence |
|---|---|---|
| mut Group | 14 | 5 |
| mut Pool | 8 | 6 |
| wild type Group | 9 | 9 |
| wild type Pool | 3 | 15 |

Sequencing of a number of these low virulence clones confirms that they are Cry toxin "cheaters", strains which have lost one or both of the large bacterial plasmid on which the majority of Cry toxin genes are encoded (as shown in Table 2 below).

**Table 2 (Genetic characterization of evolved clones (by short-read whole genome sequencings) revealed different patterns of loss for the B. t. kurstaki Cry toxin bearing plasmids. 24 clones from each treatment group were screened with PCR.)**

| Treatment | loss of both Cry plasmids (pKur2 / pBMB299 and (pKur6 / pBMB65) | loss of Cry megaplasmid (pKur2) only |
|---|---|---|
| mut Group | 0 | 0 |
| mut Pool | 1 | 5 |
| wildtype Group | 1 | 3 |
| wildtype Pool | 7 | 8 |

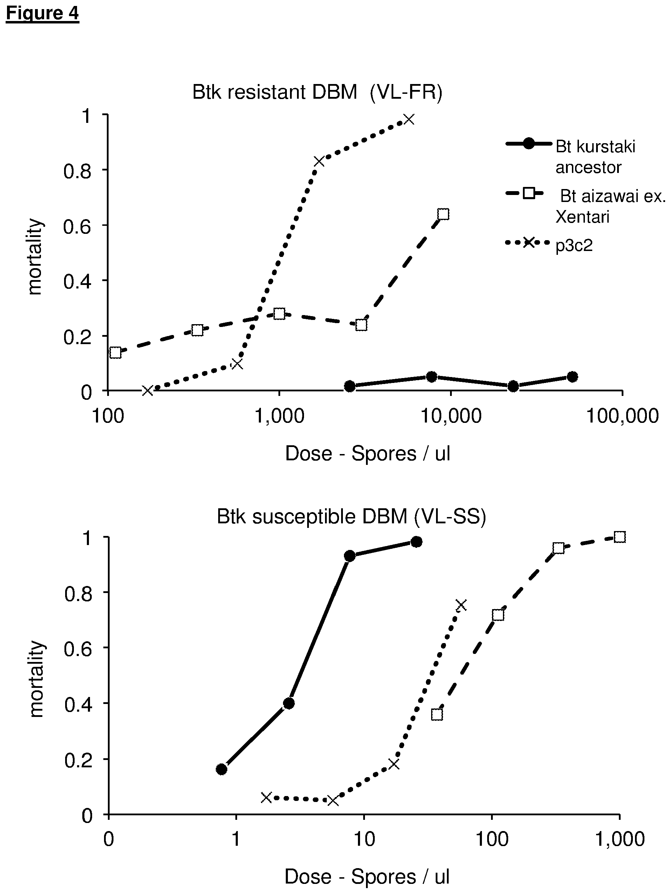

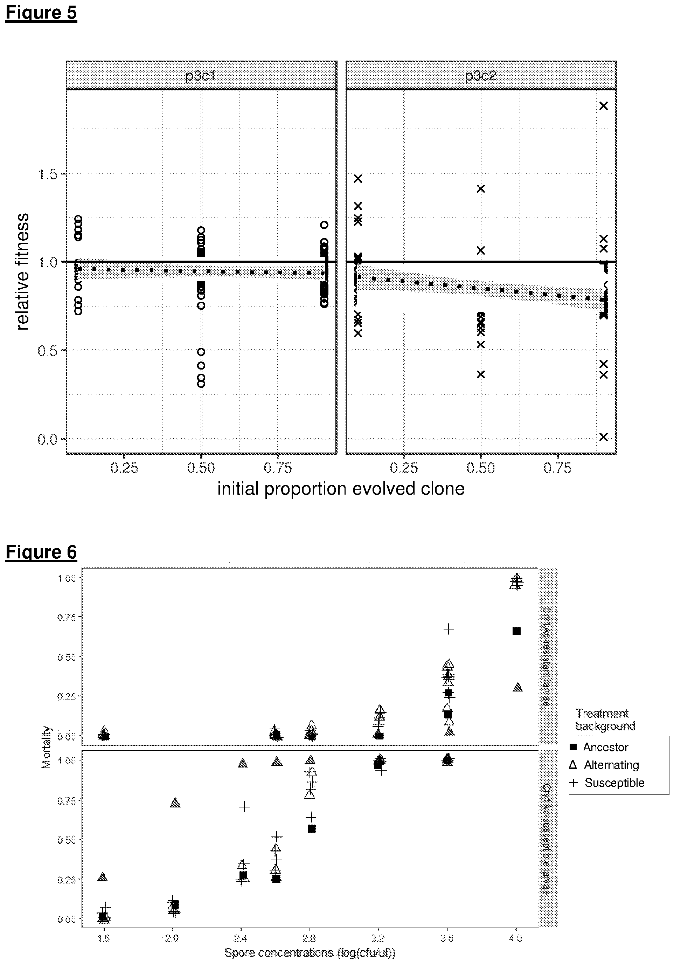

In addition to this broad characterization of a large number of clones, a subset of clones of interest was characterized in more detail in Table 3 below. Notably, several mutants were identified with increases in killing power (i.e. a reduction in LC50) of greater than an order of magnitude. For some mutators (*mgd6*), and indeed for the lineage data in Figure 4, there was substantial variation in virulence between assays, which is likely to be related to instability of mutator genotypes. Figure 5 shows the efficacy of best performing wild type clone (p3c2) against *B. t. kurstaki* resistant and susceptible *P. xylostella* in comparison to its ancestor and an alternative commerical *Bt* isolate (XenTari) used to target resistant larvae. Data are raw 5 day mortality from bioassays (*n* = 60 larvae per dose). Detailed assays of the best performing clone, p3c2, showed that it suffered some reduction in virulence against the *Bt* susceptible line VL-SS. However, it performed as well, or better, than the currently available *Bt* strain (XenTari) used to target *Cry1Ac resistant diamondback moth* (see Figure 4).

**Table 3. Detailed clone level variation in virulence (LC50s) - measured as log₁₀ transformed spore dose / colony forming units µl⁻¹ for a selection of high performing clones with increased virulence relative to their ancestor.**

| Clone | Genetic background | LC50 (SE) | Increase in virulence relative to ancestor |
|---|---|---|---|
| ancestral *B. t. kurstaki* 7.1.o | wild-type | 4.98 (0.056) | - |
| p3c1 | wild-type | 3.93 (0.02) | 11 |
| p3c2 | wild-type | 2.67 (0.024) | 203 |
| p3c3 | wild-type | 4.19 (0.026) | 6.1 |
| mga3 | mutator | 3.73 (0.055) | 17.8 |
| mgab2 | mutator | 4.32 (0.04) | 4.6 |
| mgd6 | mutator | 3.6 (0.58) | 24.0 |
| mgc5 | mutator | 4.4 (0.11) | 3.8 |

In addition to sequencing the relative fitness of two of the high virulence clones was investigated with respect to their evolved ancestor (p3c1 and p3c2 (*Bacillus thuringiensis kurstaki* NCTC (provisional accession number) 17080301 and *Bacillus thuringiensis kurstaki* NCTC (provisional accession number) 17080302 respectively)). Since group selection has the potential to increase the frequency of traits that are costly to individuals it was expected that the protocols may have allowed identification of clones that are more effective at killing hosts than their ancestors but which have reduced competitive fitness in insects, potentially because they have evolved greater levels of investment in production of virulence factors. As predicted, clone p3c2 was substantially less fit in terms of competitive fitness with respect to its ancestor (*t* = - 4.2, *P* < 0.0001); while both clones showed fitness that declined as their initial frequencies increased (*t* = -2.3, *P* = 0.022) (see Figure 5), a pattern consistent with social interactions and the costs of producing secreted virulence factors in *B. thuringiensis* (21, 28). Figure 5 shows the relative fitness of selected evolved clones with increased virulence relative to ancestor across a range of initial frequencies. Data are based on the ratio of estimated Malthusian parameters.

### Passage experiment 2

The second passage experiment had several additional aims. First, to repeat the group selection experimental protocol with a different bacterial genotype (here *B. t. entomocidus*) and second to test whether the virulence could be improved of a pathogen against a relatively susceptible host. We chose *B. t. entomocidus* (Bacillus Genetic Stock Centre strain 4I4) as a target, as it is as effective against Cry 1Ac diamondback moth larvae (LC50 ≈490 cfu/µl) (Figure 6) although not as pathogenic as *B. t. kustaki.* Resistance to Cry1Ac also provides some cross-resistance to this *B. t. entomocidus* strain (conferring an of LC50 7900 cfu/µl). Here we also set out to test whether a mutagenic agent (0.02% ethyl methanesulfonate (EMS)) could be used in lieu of mutator strains in selection to provide addition genetic variation for selection to act upon (see below). Finally, we had two specific evolutionary hypotheses to evaluate: we tested whether diversification of the host genotypes in selection lines could be used to generally improve virulence against multiple insect genotypes; this was tested by alternately passaging *Bt* lineages in Cry1Ac susceptible and resistant populations. Finally, an additional treatment tested whether increased responsiveness to selection could be gained by co-evolving both insect hosts and bacterial pathogen, as opposed to evolving pathogens against a host with a constant genetic background.

There were therefore four treatments in this selection experiment, all of which used a chemical mutagen:
1. Selection in Cry1Ac-susceptible larvae;
2. Selection in Cry1Ac resistant larvae;
3. Alternating selection in susceptible and resistant larvae;
4. Coevolution treatment, here larval populations that were heterogeneous for the Cry1Ac-resistance allele (at 10% initial frequency) were used to initiate passage. In each independent replicate, larvae that survived an infection from *Bt* under selection (ca. 400 per replicate) were reared on to adult stages to provide larvae for the next passage.

The overall structure of this selection experiment followed the group selection design in experiment 1 (Figure 1). For each passage round there were 4 treatments, each of 4 replicates of 10 subpopulations. Once mortality had reached >20%, cadavers from the 5 subpopulations with the highest/earliest mortality were kept and spores recovered from them for the next passage round. Spore inocula were adjusted between rounds to try and maintain -20% mortality.

### Mutagenic protocol

The protocol differed from previous ones as during an in-media growth phase a chemical mutagen was added, with the aim of increasing variation for selection. In practice, selected cadavers were mashed and incubated in 100µl of LB at 30°C for 3hrs, then 0.2% ethyl methanesulfonate (EMS) added and incubated for a further 3hrs. The EMS mutagen was then removed via centrifuging and resuspension, and spores the produced by adding the resuspension to 1ml HCO per subpopulation (with selective erythromycin) and incubating at 30°C for 6 days. Preliminary work with EMS identified a concentration that produced the greatest number of rifampicin resistant mutants without significant cell death, and the chosen 0.2% concentration was then used as described above.

After the final (5^{th}) round of selection, spores from each selected subpopulation in each replicate were bio-assayed against resistant and susceptible larvae (and the subsequent generation of co-evolved larvae for the resistant and co-evolution treatments) at a range of spore dilutions. Spore dilutions were varied depending on treatment and larvae in the bioassay to cover from 0% to 100% mortality, and dilutions were refined in a 2^{nd} round of bioassays to better achieve this. The ancestral *Bt entomocidus* strain was also included in all assays for comparison. Spores were grown in HCO for all endpoint bioassays.

### Results - passage experiment 2.

Here we investigated whether our use of a mutagen could also produce significant increases in virulence; whether specialized adaptation to one Cry1Ac resistant insect genotype could be offset by selecting pathogens in alternating insect genotypes and finally whether co-evolving insects and pathogens could accelerate the evolution of resistance.

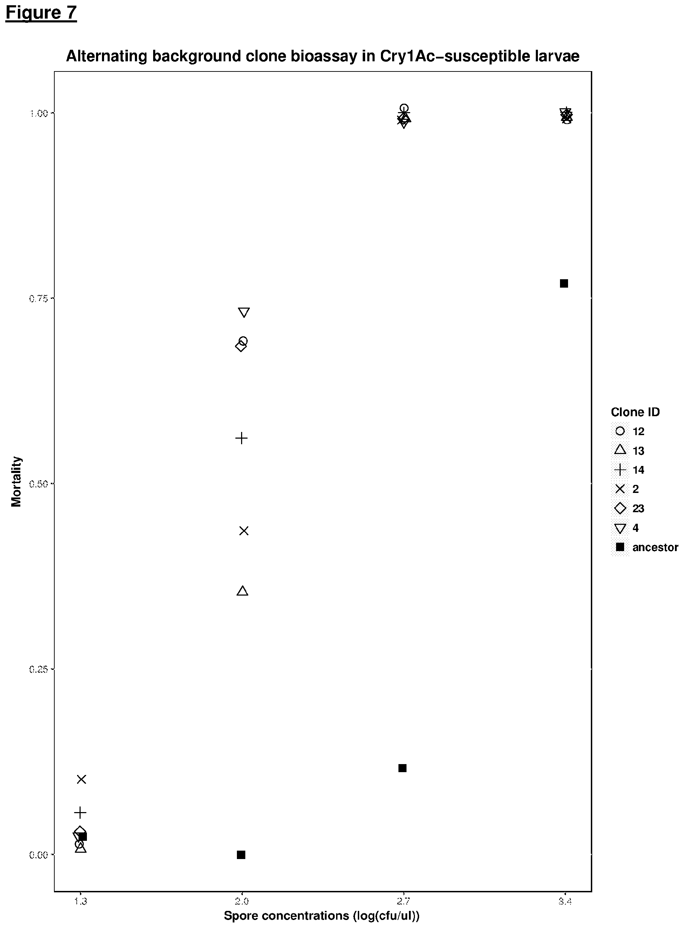

*Bt entomocidus* in the susceptible larvae and alternating treatment evolved significantly increased virulence to both Cry1Ac-resistant and susceptible hosts (mixed model binomial errors, χ² = 6.67, df = 2, *P* = 0.036) (Figure 6). However, gains in virulence tended to be relatively modest and resulted only in the halving of LC50s; with the exception of replicate 3 in the alternating treatment (shaded triangles in Figure 6), this a had 10-fold reduction in LC50 in susceptible insects (LC50 = 53.7 cfu / µl ) (Figure 6). Multiple individual clones from this lineage shared similar increases in virulence in Cry1Ac susceptible larvae relative to their ancestor (Figure 7). While this demonstrated that use of a chemical mutagenic could facilitate improvement of virulence, the alternating exposure to different genetic background did not lead to avoidance of trade-offs: gains in virulence in targeting Cry1Ac susceptible larvae were accompanied by reduction in virulence against Cry1Ac resistant larvae (Figure 6; LC50 of replicate 3 in resistant insects 26,900 cfu/ µl). Note that other evolved lines showed a consistent halving of LC50s for both resistant and susceptible larvae.

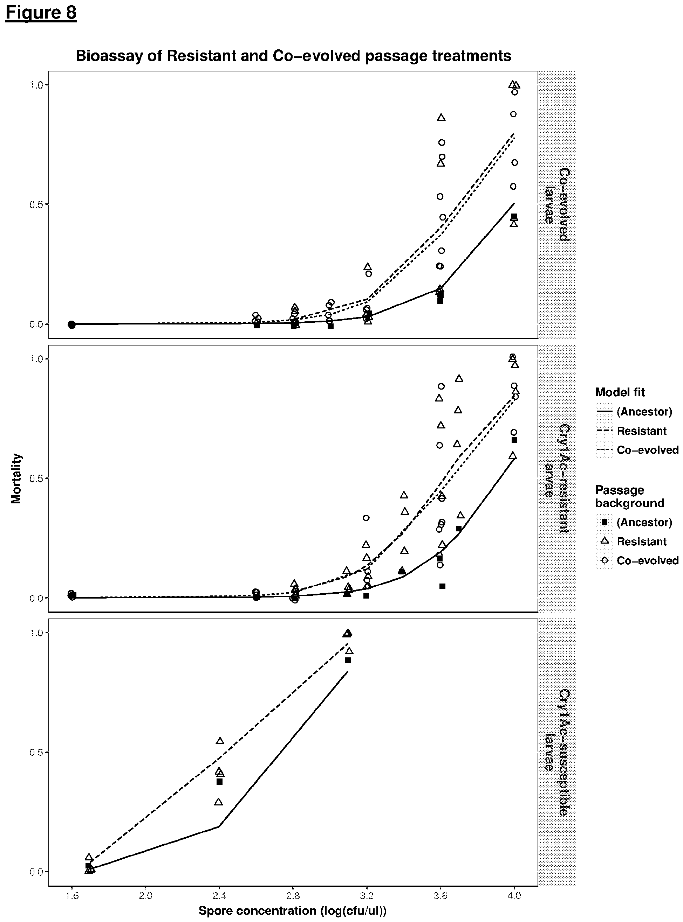

*Bt entomocidus* was also selected to increase virulence against a constant Cry1Ac resistant insect genetic background, and in a co-evolving insect background, which increased resistance as selection for virulence proceeded. Both these selection treatments produced bacteria that were significantly more virulent than their ancestor (Figure 8: resistant larvae treatment - estimate = 1.36, SE = 0.40, *t* = 3.44, *P* = 0.00787; co-evolved larvae treatment - estimate = 1.23, SE = 0.41, *t* = 2.99, *P* = 0.0034). However, both these selection regimes produced similar gains in virulence (Figure 8) so there was no additional benefit to be gained from co-evolving larvae with pathogen in this experiment. Notably at the end of the experiment the co-evolved larvae had similar levels of resistance to *B. t. entomocidus* as the Cry1Ac resistant larvae (estimate 0.3, SE = 0.26, *t* = 1.18, *P* = 0.24). In selection treatments with resistant and co-evolving larvae, improvements in killing these genetic backgrounds did not appear to result in a trade-off; evolved lineages did not acquire reduced virulence in Cry1Ac-susceptible larvae (Figure 8).

### Toxin library selection experiment.

Here we set out to test whether group selection could isolate mutants with high virulence from a library of clones with pre-existing variation in the *Bt* toxin Cry1Ac. Selection was started with a mix of 16 clones from a library of toxin variants; each variant contained one to seven amino-acid changes in Cry1Ac. Each variant was present on a pHT315 plasmid containing the *ermB* gene conferring erythromycin resistance, transformed in *Bt* 407 Cry-background. Plasmids were maintained *in vitro* with 10ug/mL erythromycin. When grown from insect cadavers, 300µg/mL erythromycin was used to inhibit growth of Gram-negative bacteria.

The passage experiment was performed according to the group selection regime as described in Passage experiment 1 (Figure 1), using the insect strain VL-SS susceptible to Cry1Ac and an infective dose of around 30 spores/µL (chosen to produce 25-30% mortality over 2-4 days for susceptible diamondback moth). In this experiment we applied two treatments, one in which spores were produced in HCO media, as per passage experiment 1, while the second treatment used additional chemical mutagenesis as per the protocol set out in passage experiment 2 (HCO +EMS). We carried out three rounds of selection. After selection 12 clones from each selection replicate were isolated from cadavers and Cry variants were identified using PCR and Sanger sequencing (48 clones per treatment in all).

### Results - toxin library selection experiment

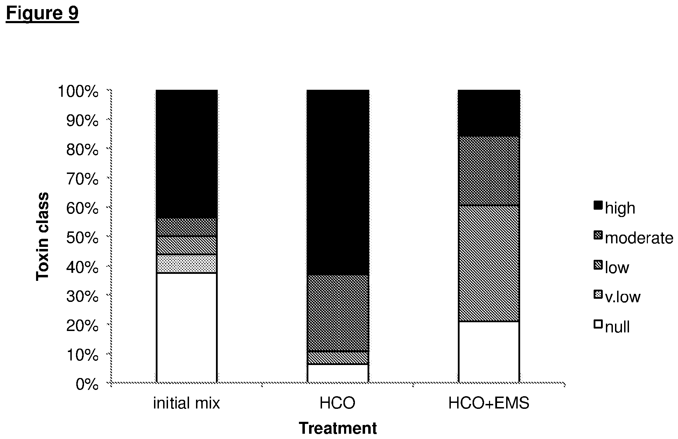

Initial bioassays indicated that 6 mutants had virulence that was indistinguishable from negative controls, *i.e.* these genotypes were null mutants that had no Cry1Ac activity (N135Q; EP8; EP26; EP23; EP14; EP15). In addition 3 mutants had suffered some moderation in virulence as a result of amino acid changes: EP13 (LC50 52 spores / µl); EP27 (32.7 spores / µl) and EP24 (23 spores / µl); these mutants are classified as having very low, low and moderate virulence respectively (see shading in Figure 9). All other mutants had amino acid changes that did not appear to affect mortality- these were classed as having "high" virulence and shared an LC50 of 12 spores / µl. Three rounds of group selection decreased the representation of Cry1Ac null mutants in both selection treatments. However, the HCO- only treatment also increasing the representation of the high virulence mutants, whereas in HCO + EMS (mutagen) treatment, mutants with low and moderate virulence increased frequencies at the expense of highly virulent mutants.

### Discussion

These experiments demonstrate a number of novel and effective steps that could be used to improve a biological pesticide based on *B. thuringiensis,* or other insect pathogens. First, it demonstrates that a combination of *in vivo* selection on mortality and *in vitro* bulking up of infectious material can be used to produce rapid changes in virulence phenotypes. Each round of infection and sporulation took under two weeks, and therefore an experienced researcher could conduct five rounds of selection in less than three months. These methods appear to be repeatable for different bacterial genetic backgrounds with different applications in mind. Notably experiments could consistently increase virulence in both highly and moderately resistant insects, although one replicate in the alternating selection regime in the *B. t. entomocidus* passage experiment did gain significantly increased virulence against susceptible insects.

Importantly, the details of the selection protocol are critical to producing good results. While previous selection experiments in both *Bt* and other pathogens have shown that pooling groups of bacteria and selecting for high population size and effective use of resources can maintain investment in virulence and produce strains with shorter time to death (*7, 11*), here it is shown that group level competition in a meta-population produced lineages with greater increases in virulence (see Figures 2, 3; Table 1) and was more effective at maintaining production of Cry toxins (see Table 2). The consequences of modifying selection design to including diverse genetic material in insect targets are not so clear-cut. Nevertheless, the improvement of 5/8 replicates in the alternating and coevolution treatments in passage experiment 2 suggests that these modifications can further refine the selective process. In these experiments co-evolving target insects with pathogens did not improve response to selection for virulence. However, this could be because resistance to Cry1Ac (in the NA-QAGE derived insects) in these experiments is monogenic and largely recessive. Individuals that are heterozygous for resistance may not have the intermediate levels of resistance that can facilitate pathogen adaptation and may appear only fleetingly in selection experiments. Coevolution regimes in target hosts with more complex modes of resistance could prove more successful. Characterization of evolved clones with increased virulence has not yet revealed any changes in sequence of Cry toxins; evolved clones also vary in their level of virulence (see Table 3) and in their reproductive rate in insects (as shown in Figure 5), suggesting that improvement has occurred via a range of mechanisms.

In addition, it has been shown that group-selection can facilitate isolation of clones with improved biocontrol potential, but which suffer some individual level cost, i.e. they have a reduced fitness in competition with their ancestor (see Figure 5). Thus this selection regime has the power to create and identify mutants that would not rise to high frequency in conventional sequential passage experiments. Since investment in virulence factors and secondary metabolites is expected to be costly for a range of pathogens, and not just *Bt,* group selection based protocols could therefore have broad potential for improving virulence in range of biological control agents. Other entomopathogenic microbes, such as fungi, which also invest in a wide range of secondary metabolites used to enhance virulence might also respond to group level selection techniques. Previous research has indicated that cheating and conflict might account for the dynamics of virulence in entomopathogenic nematodes (*35*), another important group of biological control agents. As with *Bt* these organisms, and their symbionts, secreted a wide range of protein based virulence factors important for invading, killing and digesting their insect hosts (*36*). Moreover, selection experiments indicate that treatments that limit opportunities for cheating and within host competition are better at maintaining virulence. In addition, group-level competition based on maximizing reproductive rate in cadavers did not lead to better maintenance of virulence in that study, since there appeared to be a trade-off between virulence and maximizing the production of progeny in hosts (*35*).

While the methods presented here provide a framework for the general improvement of virulence against a particular insect host, the combination of *in vivo* and *in vitro* selection could also be applied to adapted strains with good virulence characteristics to cheaper or alternative growth media, while retaining pesticidal efficacy. We have shown that protocols that rely on generating genetic variation via fixed, higher mutation rates or by chemically-induced mutagenesis can facilitate selection for increased virulence. Of the two protocols, the use of a chemical mutagen has two advantages; first it does not require the use of a genetically modified strain and second any clones produced during selection are phenotypically more stable.

We have also demonstrated proof of principle for the application of these selection techniques to pools of mutants with pre-existing genetic variation, here derived from a library of clones in which proteins of interest have been mutagenized. Notably in this experiment, there was no benefit to supplementing mutation supply with a mutagen during the course of the selection experiment.

The forgoing embodiments are not intended to limit the scope of the protection afforded by the claims, but rather to describe examples of how the invention may be put into practice.

### Biological Deposits

The application refers to the following indications of deposited biological material comprising two (2) deposits:

| | |
|---|---|
| Name: | **European Collection of Cell Cultures** |
| Address: | Public Health England Porton Down, **National Collection of Type Cultures,** PHE Culture Collections, Microbiological Services, Porton Down, Sailsbury, SP4 0JG |
| | United Kingdom |
| Date: | **03 August 2017** |
| Accession Number: | **NCTC 17080301** (provisional number) |
| Descriptor: | ***Bacillus thuringiensis kurstaki*** (p3c1) |
| Depositor: | **University of Exeter** |
| | -and- |
| Date: | **03 August 2017** |
| Accession Number: | **NCTC 17080302** (provisional number) |
| Descriptor: | ***Bacillus thuringiensis kurstaki*** (p3c2) |
| Depositor: | **University of Exeter** |

### References

1. M. Kruger, J. Van Rensburg, J. Van den Berg, Environ. Entomol. 40, 477 (2011).
2. B. E. Tabashnik, J. B. J. Van Rensburg, Y. Carrière, J. Econ. Entomol. 102, 2011 (2009).
3. T. Vaughn et al., Crop Science 45, 931 (2005).
4. M. Soberon et al., Science 318, 1640 (2007).
5. A. H. Badran et al., Nature 533, 58 (2016).
6. S. P. Diggle, A. S. Griffin, G. S. Campbell, S. A. West, Nature 450, 411 (2007).
7. A. S. Griffin, S. A. West, A. Buckling, Nature 430, 1024 (2004).
8. T. F. Cooper, PloS Biol. 5, e225 (2007).
9. R. E. Lenski, Evolution 42, 425 (1988).
10. R. D. Schulte, C. Makus, B. Hasert, N. K. Michiels, H. Schulenburg, Proceedings of the National Academy of Sciences 107, 7359 (2010).
11. J. Garbutt, M. B. Bonsall, D. J. Wright, B. Raymond, EcolLetts 14, 765 (2011).
12. M. Berling et al., Applied and Environmental Microbiology 75, 925 (2009).
13. R. Maddamsetti, P. J. Hatcher, S. Cruveiller, arXiv.org, (2012).
14. M. E. Watson, J. L. Burns, A. L. Smith, Microbiology-(UK) 150, 2947 (2004).
15. C. D. Bayliss et al., Infection and Immunity 76, 5038 (2008).
16. C. Pal, M. D. Macia, A. Oliver, I. Schachar, A. Buckling, Nature 450, 1079 (2007).
17. D. Racey, R. F. Inglis, F. Harrison, A. Oliver, A. Buckling, Evolution 642, 515 (2009).
18. J. A. G. M. de Visser, Microbiology (Reading, Engl) 148, 1247 (2002).
19. A. C. Shaver et al., Genetics 162, 557 (2002).
20. E. van Leeuwen, S. O. A. Neill, A. Matthews, B. Raymond, ISME J 9, 2328 (2015).
21. L. Zhou, L. Slamti, C. Nielsen-Leroux, D. Lereclus, B. Raymond, Current Biology 24, 2417 (2014).
22. F. Ginetti, M. Perego, A. M. Albertini, A. Galizzi, Microbiology 142, 2021 (1996).
23. N. Colegrave, S. Collins, Heredity 100, 464 (2008).
24. S. West, S. Diggle, A. Buckling, A. Gardner, A. Griffin, Annu. Rev. Ecol. Syst 38, 53 (2007).
25. S. A. West, A. Buckling, Proc. R. Soc. Lond. Ser. B-Biol. Sci. 270, 37 (2003).
26. J. B. Xavier, K. R. Foster, Proc. Natl. Acad. Sci. U. S. A. 104, 876 (2007).
27. G. J. Velicer, L. Kroos, R. E. Lenski, Nature 404, 598 (2000).
28. B. Raymond, S. A. West, A. S. Griffin, M. B. Bonsall, Science 337, 85 (2012).
29. D. Lereclus, M. Vallade, J. Chaufaux, O. Arantes, S. Rambaud, Biotechnology 10, 418 (1992).
30. F. Hunter, N. E. Crook, P. F. Entwistle, in Viruses as pathogens for the control of insects., J. M. L. Grainger, Ed. (Academic Press, New York, 1984), pp. 323-347.
31. S. W. Baxter et al., Genetics 189, 675 (2011).
32. M. M. Lecadet, M. O. Blondel, J. Ribier, J Gen Microbiol 121, 203 (1980).
33. D. Zerbino, E. Birney, Genome Research 18, 821 (2008).
34. M. Swain et al., Nature Protocols 7, 1260 (2012).
35. D. Shapiro-Ilan, B. Raymond, Evol Appl 9, 462 (2016).
36. I. Eleftherianos, S. Joyce, R. H. ffrench-Constant, D. J. Clarke, S. E. Reynolds, Parasitology 137, 1695 (2010).

## Claims

1. A *Bacillus thuringiensis* strain wherein the *B. thuringiensis* strain comprises *B. thuringiensis kurstaki* NCTC 17080301 or *B. thuringiensis kurstaki* NCTC 17080302.

2. A non-therapeutic use of the *B. thuringiensis* strain as defined in claim 1 as a pesticide, optionally in the control of one or more of the following: aphids, other Hemipteran pests, thrips, Lepidopteran larvae, Dipteran larvae, Coleopteran larvae, spider mites, locusts, crickets, ants, cockroaches, flies, wasps, termites woodworm, wood ants, bookworms, silverfish, carpet beetles, clothes moths, gypsy moths, chiggers, *Sarcoptes scabiei,* ticks, mites, lice, fleas, bed bugs, mosquitoes, tsetse flies, kissing bugs, root-knot nematode, soybean cyst nematode, potato cyst nematode, slugs, snails and the diamondback moth *Plutella xylostella* and its larvae.

3. Use of the *B. thuringiensis* strain as a pesticide according to claim 2 wherein the pesticide is applied to, near to, or in the soil of, cruciferous plants.

4. A composition comprising the *B. thuringiensis* strain according to any preceding claim.

5. The composition according to claim 4 wherein the *B. thuringiensis* strain is in the form of spores, crystals, and/or wherein the composition is in the form of a solid or a liquid.

6. The composition according to claims 4 to 5 wherein the composition further comprises one or more of the following ingredients: wetting agents, thickeners, viscosity modifying agents, stabilisers and surfactant and a dispersing medium comprising water or edible oils.

7. A non-therapeutic use of the composition according to claims 4 to 6 as a pesticide, optionally wherein the composition is for applying to plants, soil surface and/or into the soil itself by spraying, spreading or sprinkling.

8. A method for improving virulence of microbial pesticides comprising:
(a) splitting a population of pests into subpopulations;
(b) exposing the subpopulations of pests to one or more microbial pesticides, wherein the microbial pesticide has been exposed to a mutagen and wherein the microbial pesticide comprises a bacterium mutated by inactivation of the *mutS* gene;
(c) selecting pest cadavers from the subpopulations with highest mortality;
(d) inoculating sporulation media with the pest cadavers so as to provide sufficient readily quantifiable inoculum for a subsequent round of infection;
(e) purifying and quantifying microbial spores from the selected cadavers so as to identify one or more microbial pesticides having improved virulence.

9. The method according to claim 8 further comprising using microbial spores from step (e) to infect one or more second pest populations.

10. The method according to claims 8 or 9 wherein steps (a) to (e) are repeated at least once, and optionally, the microbial pesticide is repeatedly exposed to the mutagen.

11. The method according to claims 8 to 10, wherein the cadavers are selected during step (c) on the basis of total mortality of the pest population reaching in the range of 25-30%.

12. The method according to claims 8 to 11 wherein a single clone is selected from the purified microbial spores of step (e) to infect further pest populations, and optionally, wherein a single clone is selected for further infection at least twice per 5 passages.

13. method according to claims 9 to 12 wherein the one or more pest populations comprise genetically variable populations.

14. The method according to claims 9 to 13 wherein the pest populations comprises;
(a) different levels of resistance to the microbial pesticides or one or more toxic compounds produced by the microbial pesticides, or
(b) an intermediate level of resistance to the microbial pesticides or one or more toxic compounds produced by the microbial pesticides.

15. The method according to claims 8 to 14 wherein the method is for use in providing a library of microbial pesticides.

## Patentansprüche

1. *Bacillus thuringiensis-Stamm,* wobei der *B*. *thuringiensis-Stamm B. thuringiensis kurstaki* NCTC 17080301 oder *B. thuringiensis kurstaki* NCTC 17080302 umfasst.

2. Nicht-therapeutische Verwendung des *B*. *thuringiensis-Stamms,* wie in Anspruch 1 definiert, als ein Pestizid, gegebenenfalls zur Kontrolle von einem oder mehreren der Folgenden: Blattläuse, andere Hemiptera-Schädlinge, Thripe, Lepidoptera-Larven, Diptera-Larven, Coleoptera-Larven, Spinnmilben, Heuschrecken, Grillen, Ameisen, Schaben, Fliegen, Wespen, Termiten Holzwurm, Waldameise, Bücherwürmer, Silberfischchen, Teppichkäfer, Kleidermotten, Schwammspinner, Sandflöhe, *Sarcoptes scabiei,* Zecken, Milben, Läuse, Flöhe, Bettwanzen, Moskitos, Tsetsefliegen, Raubwanzen "Kissing bug", Wurzelgallennematode, Sojabohnenzystennematode, Kartoffelzystennematode, Nacktschnecken, Schnecken und die Kohlmotte *Plutella xylostella* und ihre Larven.

3. Verwendung des *B. thuringiensis-Stamms* als ein Pestizid nach Anspruch 2, wobei das Pestizid auf, in der Nähe oder im Boden von Kreuzblütlern angewendet wird.

4. Zusammensetzung, umfassend den *B. thuringiensis-*Stamm nach einem der vorhergehenden Ansprüche.

5. Zusammensetzung nach Anspruch 4, wobei der *B*. *thuringiensis-*Stamm in Form von Sporen, Kristallen vorliegt, und/oder wobei die Zusammensetzung in Form eines Feststoffs oder einer Flüssigkeit vorliegt.

6. Zusammensetzung nach Ansprüchen 4 bis 5, wobei die Zusammensetzung ferner ein oder mehrere der folgenden Bestandteile umfasst: Netzmittel, Verdickungsmittel, viskositätsmodifizierende Mittel, Stabilisatoren und Tenside und ein Dispersionsmedium, das Wasser oder Speiseöle umfasst.

7. Nicht-therapeutische Verwendung der Zusammensetzung nach Ansprüchen 4 bis 6 als ein Pestizid, gegebenenfalls wobei die Zusammensetzung für die Anwendung auf Pflanzen, Bodenoberfläche und/oder in den Boden selbst durch Sprühen, Verteilen oder Besprühen bestimmt ist.

8. Verfahren zur Verbesserung der Virulenz von mikrobiellen Pestiziden, umfassend:
(a) Aufteilen einer Population von Schädlingen in Subpopulationen;
(b) Aussetzen der Subpopulationen von Schädlingen eines oder mehreren mikrobiellen Pestiziden,
wobei das mikrobielle Pestizid einem Mutagen ausgesetzt wurde und wobei das mikrobielle Pestizid ein Bakterium umfasst, das durch Inaktivierung des *mutS*-Gens verändert wurde;
(c) Auswählen von Schädlingskadavern aus den Subpopulationen mit der höchsten Mortalität;
(d) Beimpfen des Sporulationsmediums mit den Schädlingskadavern, um ausreichend leicht quantifizierbares Inokulum für eine nachfolgende Infektionsrunde bereitzustellen;
(e) Reinigen und Quantifizieren der mikrobiellen Sporen aus den ausgewählten Kadavern, um ein oder mehrere mikrobielle Pestizide zu identifizieren, die verbesserte Virulenz aufweisen.

9. Verfahren nach Anspruch 8, ferner umfassend die Verwendung von mikrobiellen Sporen aus Schritt (e), um eine oder mehrere zweite Schädlingspopulationen zu infizieren.

10. Verfahren nach Anspruch 8 oder 9, wobei Schritte (a) bis (e) zumindest einmal wiederholt werden, und gegebenenfalls, das mikrobielle Pestizid wiederholt dem Mutagen ausgesetzt wird.

11. Verfahren nach Ansprüchen 8 bis 10, wobei die Kadaver während Schritt (c) auf der Grundlage der Gesamtmortalität der Schädlingspopulation ausgewählt werden, die im Bereich von 25-30 % liegt.

12. Verfahren nach Ansprüchen 8 bis 11, wobei ein einzelner Klon aus den gereinigten mikrobiellen Sporen aus Schritt (e) ausgewählt wird, um ferner Schädlingspopulationen zu infizieren, und gegebenenfalls, wobei ein einzelner Klon zumindest zweimal pro 5 Durchgänge für eine weitere Infektion ausgewählt wird.

13. Verfahren nach Ansprüchen 9 bis 12, wobei die eine oder mehreren Schädlingspopulationen genetisch variable Populationen umfassen.

14. Verfahren nach Ansprüchen 9 bis 13, wobei die Schädlingspopulationen Folgendes umfassen:
(a) unterschiedliche Resistenzgrade gegenüber den mikrobiellen Pestiziden oder einer oder mehreren toxischen Verbindungen, die durch die mikrobiellen Pestizide hergestellt werden, oder
(b) einen mittleren Resistenzgrad gegenüber den mikrobiellen Pestiziden oder einer oder mehreren toxischen Verbindungen, die durch die mikrobiellen Pestizide hergestellt werden.

15. Verfahren nach Ansprüchen 8 bis 14, wobei das Verfahren zur Verwendung bei der Bereitstellung einer Bibliothek von mikrobiellen Pestiziden bestimmt ist.

## Revendications

1. Souche de *Bacillus thuringiensis,*
la souche de *B. thuringiensis* comprenant *B. thuringiensis kurstaki* NCTC 17080301 ou *B. thuringiensis kurstaki* NCTC 17080302.

2. Utilisation non thérapeutique de la souche de B. *thuringiensis* telle que définie dans la revendication 1 en tant que pesticide, éventuellement dans la lutte contre l'un ou plusieurs parmi ce qui suit : pucerons, autres hémiptères nuisibles, thrips, larves de lépidoptères, larves de diptères, larves de coléoptères, tétranyques, sauterelles, grillons, fourmis, cafards, mouches, guêpes, termites, vers de bois, fourmis des bois, vers de livres, lépismes, coléoptères des tapis, mites des vêtements, spongieuses, aoûtats, *Sarcoptes scabiei,* tiques, acariens, poux, puces, punaises de lit, moustiques, mouches tsé-tsé, punaises baissières, nématode à galles, nématode à kystes du soja, nématode à kystes de la pomme de terre, limaces, escargots et la teigne des crucifères *Plutella xylostella* et ses larves.

3. Utilisation de la souche de *B. thuringiensis* en tant que pesticide selon la revendication 2, le pesticide étant appliqué sur des, à proximité de, ou dans le sol de, végétaux crucifères.

4. Composition comprenant la souche de *B. thuringiensis* selon une quelconque revendication précédente.

5. Composition selon la revendication 4, la souche de B. thuringiensis étant sous la forme de spores, de cristaux et/ou la composition étant sous la forme d'un solide ou d'un liquide.

6. Composition selon les revendications 4 à 5, la composition comprenant en outre un ou plusieurs des ingrédients suivants : des agents mouillants, des épaississants, des agents de modification de la viscosité, des stabilisants et un tensioactif et un milieu de dispersion comprenant de l'eau ou des huiles comestibles.

7. Utilisation non thérapeutique de la composition selon les revendications 4 à 6 en tant que pesticide, éventuellement
la composition étant pour une application sur des végétaux, la surface du sol et/ou dans le sol luimême par pulvérisation, étalement ou aspersion.

8. Procédé pour l'amélioration de la virulence de pesticides microbiens comprenant :
(a) la division d'une population d'organismes nuisibles en sous-populations ;
(b) l'exposition des sous-populations d'organismes nuisibles à un ou plusieurs pesticides microbiens, le pesticide microbien ayant été exposé à un mutagène et le pesticide microbien comprenant une bactérie mutée par inactivation du gène *mutS ;*
(c) la sélection de cadavres d'organismes nuisibles parmi les sous-populations dotées d'une mortalité la plus élevée ;
(d) l'inoculation de milieux de sporulation avec les cadavres d'organismes nuisibles de sorte à fournir suffisamment d'inoculum facilement quantifiable pour un tour subséquent d'infection ;
(e) la purification et la quantification de spores microbiens à partir des cadavres sélectionnés de sorte à identifier un ou plusieurs pesticides microbiens ayant une virulence améliorée.

9. Procédé selon la revendication 8 comprenant en outre une utilisation de spores microbiens de l'étape (e) pour infecter une ou plusieurs deuxièmes populations d'organismes nuisibles.

10. Procédé selon la revendication 8 ou 9, les étapes (a) à (e) étant répétées au moins une fois, et éventuellement, le pesticide microbien étant exposé de manière répétée au mutagène.

11. Procédé selon les revendications 8 à 10, les cadavres étant sélectionnés pendant l'étape (c) sur la base du fait que la mortalité totale de la population d'organismes nuisibles atteint la plage de 25 à 30 %.

12. Procédé selon les revendications 8 à 11, un unique clone étant choisi parmi les spores microbiens purifiés de l'étape (e) pour infecter d'autres populations d'organismes nuisibles, et éventuellement, un unique clone étant choisi pour une infection supplémentaire au moins deux fois par 5 passages.

13. Procédé selon les revendications 9 à 12, la ou les populations d'organismes nuisibles comprenant des populations génétiquement variables.

14. Procédé selon les revendications 9 à 13, les populations d'organismes nuisibles comprenant :
(a) des niveaux différents de résistance aux pesticides microbiens ou à un ou plusieurs composés toxiques produits par les pesticides microbiens, ou
(b) un niveau intermédiaire de résistance aux pesticides microbiens ou à un ou plusieurs composés toxiques produits par les pesticides microbiens.

15. Procédé selon les revendications 8 à 14, le procédé étant pour une utilisation dans la fourniture d'une bibliothèque de pesticides microbiens.
